Europäisches Patentamt

European Patent Office

Office européen des brevets

(19)

(11) **EP 1 017 730 B1**

(12) **EUROPÄISCHE PATENTSCHRIFT**

(45) Veröffentlichungstag und Bekanntmachung des Hinweises auf die Patenterteilung:
**26.03.2003 Patentblatt 2003/13**

(21) Anmeldenummer: **98948856.4**

(22) Anmeldetag: **21.08.1998**

(51) Int Cl.$^7$: **C08F 10/06**

(86) Internationale Anmeldenummer:
**PCT/EP98/05334**

(87) Internationale Veröffentlichungsnummer:
**WO 99/011678 (11.03.1999 Gazette 1999/10)**

(54) **SPRITZGIESSARTIKEL AUS METALLOCEN-POLYPROPYLEN**

INJECTION MOULDING ARTICLES MADE OF METALLOCENE AND POLYPROPYLENE

ARTICLES MOULES PAR INJECTION CONSTITUES D'UN ALLIAGE ORGANOMETALLIQUE ET DE PROPYLENE

(84) Benannte Vertragsstaaten:
**BE DE ES FR GB IT NL**

(30) Priorität: **01.09.1997 DE 19738051**

(43) Veröffentlichungstag der Anmeldung:
**12.07.2000 Patentblatt 2000/28**

(73) Patentinhaber: **Basell Polyolefine GmbH**
**50389 Wesseling (DE)**

(72) Erfinder:
- **GRASMEDER, John, Russell**
  **D-67246 Dirmstein (DE)**
- **OVERTHUN, Klaus**
  **D-67136 Fu gönheim (DE)**

(56) Entgegenhaltungen:

| | |
|---|---|
| **EP-A- 0 444 474** | **EP-A- 0 593 888** |
| **EP-A- 0 596 379** | **EP-A- 0 622 410** |
| **EP-A- 0 645 401** | **EP-A- 0 751 215** |
| **EP-A- 0 878 567** | **WO-A-94/07951** |
| **WO-A-96/32441** | **WO-A-97/08238** |
| **WO-A-97/19980** | **WO-A-97/19991** |
| **WO-A-97/27591** | **WO-A-97/31035** |
| **WO-A-97/47662** | **DE-A- 19 727 065** |
| **DE-U- 29 603 840** | **DE-U- 29 700 263** |
| **JP-A- 51 026 986** | **US-A- 4 637 222** |

- **PATENT ABSTRACTS OF JAPAN vol. 097, no. 008, 29. August 1997 & JP 09 111058 A (MITSUBISHI CHEM CORP), 28. April 1997 -& EP 0 857 754 A (MITSUBISHI CHEMICAL CORPORATION) 12. August 1998**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft die Verwendung von Homopolymerisaten des Propylens oder Copolymerisaten des Propylens mit $C_2$ bis $C_{10}$-Alk-1-enen, die durch die Polymerisation der entsprechenden Monomeren mit Metallocenkatalysatoren erhältlich sind zur Herstellung von Spritzgieß-Artikeln für den Audio-, Video- oder EDV-Bereich.

Kunststoffe, insbesondere Olefinpolymerisate, werden mit der Spritzgießtechnik zu Formkörpern verarbeitet.

Derartige Formkörper, oder die ihnen zugrunde liegenden Polymere, haben jedoch Nachteile.

Formkörper mit hoher Transparenz, beispielsweise aus Randomcopolymerisaten des Propylens mit anderen Olefinen, haben üblicherweise eine unbefriedigende Steifigkeit, ausgedrückt durch das E-Modul nach ASTM D882.

Formkörper mit hoher Steifigkeit, beispielsweise aus Homopolymerisaten des Propylens, haben andererseits in der Regel eine ungenügende Transparenz, gemessen nach ASTM D1003.

Viele der zur Zeit verfügbaren Polyolefine, die zur Anwendung im Spritzgießen kommen, haben noch unbefriedigende organoleptische Eigenschaften (unangenehmen Geruch und/oder Geschmack des gefertigten Artikels).

EP-A 593 888 beschreibt die Verwendung von Homopolymerisaten des Propylens, welche durch Metallocen-Katalysatoren hergestellt wurden, zur Herstellung von Formkörpern nach der Spritzgußtechnik.

WO 97/27591 beschreibt die Herstellung von Behältern für CD's und CD-ROM's aus Polypropylen durch Falten eines aus einer extrudierten Platte erhaltenen Zuschnitts.

Es bestand daher die Aufgabe, Polymerisate zu entwickeln, die hohe Transparenz bei gleichzeitiger guter Steifigkeit in sich vereinen, und die zudem noch eine geringe Geruchs- und/oder Geschmacksbelästigung (gute organoleptische Eigenschaften) geringe xylollösliche Anteile, vorzugsweise weniger als 1,5 Gew.-%, aufweisen.

Demgemäß wurde die Verwendung von Homopolymerisaten des Propylens oder Copolymerisate des Propylens mit $C_2$- bis $C_{10}$-Alk-1-enen, die durch die Polymerisation der entsprechenden Monomeren mit Metallocenkatalysatoren erhältlich sind, zur Herstellung von Spritzgießartikel, wie in den Patentansprüchen definiert, gefunden.

Die erfindungsgemäßen Polymerisate des Propylens sind Propylenhomopolymere oder Copolymere aus Propylen und einem Alk-1-en oder mehreren Alk-1-enen, ausgewählt aus der Gruppe bestehend aus Ethylen, 1-Buten, 1-Penten, 1-Hexen, 1-Hepten, 1-Octen, 1-Nonen, 1-Decen oder 4-Methyl-1-penten, oder Mischungen dieser Polymeren, wobei die Mischungsverhältnisse nicht kritisch sind. Als Copolymere werden im allgemeinen statistische Copolymere verstanden.

Die Propylenhomopolymere sind im wesentlichen isotaktisch.

Die Homopolymerisate des Propylens oder Copolymerisate des Propylens mit $C_2$- bis $C_{10}$-Alk-1-enen, mit der voranstehend beschriebenen Zusammensetzung und Struktur, werden durch die Polymerisation der entsprechenden Monomeren mit Metallocenkatalysatoren erhalten. Im folgenden werden derartige Propylenpolymere "erfindungsgemäße Propylenhomo und -copolymere" genannt.

Unter Metallocenkatalysatoren sind hierin Stoffe zu verstehen, die im allgemeinen durch die Kombination einer Übergangsmetallverbindung oder mehrerer Übergangsmetallverbindungen, vorzugsweise des Titans, Zirkoniums oder Hafniums, die mindestens einen Liganden enthält, der im weitesten Sinne ein Derivat des Cyclopentadienylliganden ist, mit einem Aktivator, auch Cokatalysator oder metalloceniumionenbildende Verbindung genannt, entsteht und im allgemeinen gegenüber den beschriebenen Monomeren polymerisationsaktiv sind. Derartige Katalysatoren sind beispielsweise in EP-A 0 545 303, EP-A 0 576 970 und EP-A 0 582 194 beschrieben.

Gut geeignete Katalysatoren sind beispielsweise in WO 97/19980, Seite 3, Zeile 16 bis Seite 11, Zeile 17 beschrieben.

Ganz besonders bevorzugte Metallocenkomponenten der Katalysatorsysteme sind

rac.-Dimethylsilylenbis(2-methylbenzindenyl)zirkondichlorid

rac.-Dimethylsilylenbis(2-ethylbenzindenyl)zirkondichlorid

rac.-Dimethylsilylenbis(2-methylindenyl)zirkondichlorid

rac.-Dimethylsilylenbis(2,4-dimethylindenyl)zirkondichlorid

rac.-Dimethylsilylenbis(2,4,7-trimethylindenyl)zirkondichlorid

rac.-Dimethylsilylenbis(2-methyl-4-isopropylindenyl)zirkondichlorid

rac.-Dimethylsilylenbis(2-methyl-4,6-diisopropylindenyl)zirkondichlorid

rac.-Dimethylsilylenbis(2-methyl-4-phenylindenyl)zirkondichlorid

rac.-Dimethylsilylenbis(2-ethyl-4-phenylindenyl)zirkondichlorid

rac.-Dimethylsilylenbis(2-methyl-4-naphthylindenyl)zirkondichlorid

rac. -Ethylenbis (2-methylbenzindenyl)zirkondichlorid

rac. -Ethylenbis(2-ethylbenzindenyl)zirkondichlorid

rac. -Ethylenbis(2-methylindenyl)zirkondichlorid

rac. -Ethylenbis(2,4-dimethylindenyl)zirkondichlorid

rac. -Ethylenbis(2,4,7-trimethylindenyl)zirkondichlorid

rac. -Ethylen(2-methyl-4-isopropylindenyl)zirkondichlorid

rac. -Ethylenbis(2-methyl-4,6-diisopropylindenyl)zirkondichlorid

rac. -Ethylenbis(2-methyl-4-phenylindenyl)zirkondichlorid

rac.-Ethylenbis(2-ethyl-4-phenylindenyl)zirkondichlorid

rac.-Ethylenbis(2-methyl-4-naphthylindenyl)zirkondichlorid

Die Herstellung der Propylenpolymerisate kann in den üblichen, für die Polymerisation von Olefinen verwendeten Reaktoren entweder diskontinuierlich oder vorzugsweise kontinuierlich erfolgen. Geeignete Reaktoren sind unter anderem kontinuierlich betriebene Rührkessel oder Schleifenreaktoren, wobei man gegebenenfalls auch eine Reihe von mehreren hintereinandergeschalteten Rührkesseln oder Schleifenreaktoren verwenden kann. Die Polymerisationsreaktionen lassen sich in der Gasphase in Suspension, in flüssigen und in überkritischen Monomeren oder in inerten Lösungsmitteln durchführen.

Die Polymerisationsbedingungen sind an sich unkritisch. Drücke von 100 bis 350 000 kPa, vorzugsweise 100 bis 250 000 und insbesondere 100 bis 100 000 kPa, Temperaturen von 0 bis 400°C, vorzugsweise 20 bis 250°C und insbesondere 50 bis 100°C haben sich als geeignet erwiesen.

Das mittlere Molekulargewicht der Polymeren kann mit den in der Polymerisationstechnik üblichen Methoden gesteuert werden, beispielsweise durch Zufuhr von Molekulargewichtsreglern, wie Wasserstoff, welche zu einer Reduzierung des Molekulargewichts des Polymeren führt oder durch Variation der Polymerisationstemperatur, wobei hohe Polymerisationstemperaturen üblicherweise ebenfalls zu reduzierten Molekulargewichten führen.

Die erfindungsgemäßen Propylenhomo und -copolymeren haben im allgemeinen einen Schmelzflußindex (Melt Flow Rate, MFR) gemessen bei 230°C und 2,16 kg Auflagegewicht nach DIN 53735 im Bereich von 10 bis 100, vorzugsweise im Bereich von 40 bis 80 und insbesondere im Bereich von 50 bis 65 g/10 min.

Die Molekulargewichtsverteilung der erfindungsgemäßen Propylenhomo und -copolymere Mw/Mn, bestimmt mittels GPC bei 140°C in 1,2,4-Trichlorbenzol gegen Polypropylenstandard, liegt im allgemeinen im Bereich von 1,2 bis 3,0, vorzugsweise 1,2 bis 2,5.

Sowohl das Molekulargewicht Mw, die Molekulargewichtsverteilung Mw/Mn, sowie insbesondere der MFR-Wert können auch mit der Methode des peroxidisch initiierten Abbaus eines Ausgangspolymerisats, vorteilhaft in einem Extruder, eingestellt werden. Diese Methode ist dem Fachmann bekannt.

Die erfindungsgemäßen Homopolymerisate des Propylens haben im allgemeinen einen Schmelzpunkt, bestimmt mit der Methode der Differential Scanning Calorimetrie (DSC) im Bereich von 80°C bis 170°C, vorzugsweise im Bereich von 135°C bis 165°C und insbesondere im Bereich von 140°C bis 165°C.

Die erfindungsgemäßen Copolymerisate des Propylens mit C$_2$- bis C$_{10}$-Alk-1-enen haben im allgemeinen einen Schmelzpunkt, bestimmt mit der Methode der Differential Scanning Calorimetrie (DSC) (Aufheizgeschwindigkeit 20°C/min.), im Bereich von 60°C bis 160°C, vorzugsweise im Bereich von 80 bis 150°C und insbesondere im Bereich von 100°C bis 150°C.

Der Pentadengehalt mmmm, mit anderen worten die Isotaktizität, der erfindungsgemäßen Homopolymerisate, bestimmt mit der Methode der 13C-NMR-Spektroskopie, liegt üblicherweise im Bereich von 60 % bis 99 %, vorzugsweise im Bereich von 80 % bis 98 %.

Die xylollöslichen Anteile der erfindungsgemäßen Propylenhomo und -copolymeren sind üblicherweise geringer als 1,5 Gew."%, vorzugsweise geringer als 1,0 Gew.-%.

Die genannten xylollöslichen Anteile X$_L$ wurden folgendermaßen bestimmt:

In einen 1-Liter-Dreihalskolben mit Rührer, Rückflußkühler und Thermometer wurden 500 ml destilliertes Xylol (Isomerengemisch) eingefüllt und auf 100°C erhitzt. Bei dieser Temperatur wurde das Polymere eingefüllt, anschließend auf den Siedepunkt des Xylols erhitzt und 60 min am Rückfluß gehalten. Anschließend wurde die Wärmezufuhr abgebrochen, innerhalb von 20 min mit einem Kältebad auf 5°C abgekühlt und dann wieder auf 20°C erwärmt. Diese Temperatur wurde für 30 min gehalten. Das ausgefallene Polymerisat wurde abfiltriert und von dem Filtrat exakt 100 ml in einen vorher tarierten 250-ml-Einhalskolben gefüllt. Daraus wurde das Lösungsmittel am Rotationsverdampfer entfernt. Anschließend würde der verbleibende Rückstand im Vakuumtrockenschrank bei 80°C/200 Torr für 2 h getrocknet. Nach dem Erkalten wurde ausgewogen.

Der xylollösliche Anteil ergibt sich aus

$$X_L = \frac{g \times 500 \times 100}{G \times V}$$

X$_L$ =     xylollöslicher Anteil in %

g =     gefundene Menge

G =     Produkteinwaage

V =     Volumen der eingesetzten Filtratmenge

Der chemisch gebundene Comonomeranteil der erfindungsgemäßen vorzugsweise statistischen Copolymerisate des Propylens mit C$_2$- bis C$_{10}$-Alk-1-enen, gemessen mit der Methode der 13C-NMR-Spektroskopie, liegt im allgemeinen im Bereich von 0,001 bis 35 mol-%, vorzugsweise im Bereich von 0,01 bis 15 mol-%, bezogen auf das Copolymere. Als C$_2$- bis C$_{10}$-Alken kommen insbesondere Ethylen, 1-Bute und deren Gemisch in Frage.

Ein gut geeignetes Propylenpolymerisat ist das Homopolypropylen NOVOLEN M NX 50081 der Targor GmbH (vorher BASF Aktiengesellschaft).

Die erfindungsgemäßen Formkörper (Spritzgußartikel) werden im allgemeinen mit den üblichen, dem Fachmann bekannten, Spritzgieß-Verfahren hergestellt.

Der E-Modul der erfindungsgemäßen Propylenhomo und -copolymeren, gemessen im Zugversuch nach ISO 527, liegt im allgemeinen im Bereich von 1300 bis 7500, vorzugsweise im Bereich von 1500 bis 7500.

Der Haze, als Komplementärwert für Transparenz, bestimmt nach ASTM D 1003, beträgt für die erfindungsgemäßen Propylenhomo und -copolymeren weniger als 10, vorzugsweise weniger als 8 %.

Die erfindungsgemäßen Spritzgieß-Artikel können noch die üblichen Thermoplast-Additive in den üblichen Mengen enthalten. Als Additive kommen in Frage Antistatika, Gleitmittel, wie Fettsäureamide, beispielsweise Erucasäureamid, Stabilisatoren, Neutralisationsmittel, wie Calciumstearat, Pigmente und außerdem anorganische Füllstoffe wie Talkum, Aluminiumoxid, Aluminiumsulfat, Bariumsulfat, Calciummagnesiumcarbonat, Siliciumdioxid, Titandioxid, Glasfasern sowie organische Füllstoffe wie Polyester, Polystyrol, Polyamid und halogenierte organische Polymere.

Bevorzugte Additive sind außerdem Nucleiierungsmittel, wie Talkum, Alkali- oder Aluminium-Salze von Carbon- und Alkylarylsäuren, bestimmte Polymere wie Polyvinylcylohexan oder Polycyclopenten und Polyhydroxyverbindungen, wie Sorbitol-Derivate. Besonders bevorzugt sind Sorbitol-Derivate.

Die erfindungsgemäßen Propylenhomo und -copolymeren sind gut geeignet zur Herstellung von Spritzgiessartikeln für diverse Verwendungen, wie exemplarisch im folgenden beschrieben.

Die erfindungsgemäßen Propylenhomo und -copolymeren sind geeignet für Verwendungen (Anwendungen)

im Audio/Video/Computer-Bereich

wie CD/CD-ROM Verpackungen, Cassettenhüllen (Audio/Video), Boxen für Disketten und Bänder.

## Patentansprüche

1. Verwendung von Homopolymerisaten des Propylens oder Copolymerisaten des Propylens mit $C_2$-$C_{10}$-Alk-1-enen, die durch die Polymerisation der entsprechenden Monomeren mit Metallocenkatalysatoren erhältlich sind, zur Herstellung von Spritzgießartikeln für den Audio-, Video- oder EDV-Bereich.

2. Verwendung gemäß Anspruch 1, wobei es sich bei den Spritzgießartikeln für den Audio-, Video- oder EDV-Bereich um CD- oder CD-ROM-Verpackungen handelt.

3. Verwendung gemäß Anspruch 1, wobei es sich bei den Spritzgießartikeln für den Audio-, Video- oder EDV-Bereich um Cassettenhüllen für Audio oder Video handelt.

4. Verwendung gemäß Anspruch 1, wobei es sich bei den Spritzgießartikeln für den Audio-, Video- oder EDV-Bereich um Boxen für Disketten oder Bänder handelt.

5. CD- oder CD-ROM-Verpackungen aus Homopolymerisaten des Propylens oder Copolymerisaten des Propylens mit $C_2$- $C_{10}$-Alk-lenen, die durch die Polymerisation der entsprechenden Monomeren mit Metallocenkatalysatoren erhältlich sind.

## Claims

1. The use of homopolymers of propylene or copolymers of propylene with $C_2$-$C_{10}$-alk-1-enes, which polymers are obtainable by polymerization of the corresponding monomers using metallocene catalysts, for producing injection-molded articles for the audio, video or EDP sector.

2. The use as claimed in claim 1, wherein the injection-molded articles for the audio, video or EDP sector are CD packaging or CD-ROM packaging.

3. The use as claimed in claim 1, wherein the injection-molded articles for the audio, video or EDP sector are cassette bodies for audio or video.

4. The use as claimed in claim 1, wherein the injection-molded articles for the audio, video or EDP sector are boxes for floppy disks or tapes.

5. CD packaging or CD-ROM packaging comprising a homopolymer of propylene or a copolymer of propylene with $C_2$-$C_{10}$-alk-1-enes which polymer is obtainable by polymerization of the corresponding monomers using metallocene catalysts.

## Revendications

1. Mise en oeuvre d'homopolymères du propylène ou de copolymères du propylène avec des alcènes en $C_2$ à $C_{10}$, que l'on peut obtenir par polymérisation des monomères correspondants avec des catalyseurs de métallocènes, pour la production d'articles moulés par injection destinés au domaine du son, de l'audiovisuel ou de l'informatique.

2. Mise en oeuvre selon la revendication 1, dans laquelle les articles moulés par injection destinés au domaine du son, de l'audiovisuel ou de l'informatique sont des emballages pour disques compacts ou cédérom.

3. Mise en oeuvre selon la revendication 1, dans laquelle les articles moulés par injection destinés au domaine du son, de l'audiovisuel ou de l'informatique sont des boîtiers pour cassettes de son ou audiovisuel.

4. Mise en oeuvre selon la revendication 1, dans laquelle les articles moulés par injection destinés au domaine du son, de l'audiovisuel ou de l'informatique sont des boîtiers pour disquettes ou pour bandes magnétiques.

5. Emballages de CD ou de CD-ROM constitués d'homopolymères du propylène ou de copolymères du propylène avec des alcènes en $C_2$ à $C_{10}$, que l'on peut obtenir par polymérisation des monomères correspondants avec des catalyseurs de métallocènes.